**Europäisches Patentamt**

**European Patent Office**

**Office européen des brevets**

(11) Veröffentlichungsnummer: **0 290 983 B1**

# EUROPÄISCHE PATENTSCHRIFT

(45) Veröffentlichungstag der Patentschrift: **25.01.95**

(51) Int. Cl.[6]: **A61L 25/00**, A61L 15/16

(21) Anmeldenummer: **88107362.1**

(22) Anmeldetag: **07.05.88**

(54) **Verbesserte resorbierbare Knochenwachse.**

(30) Priorität: **15.05.87 DE 3716302**

(43) Veröffentlichungstag der Anmeldung:
**17.11.88 Patentblatt 88/46**

(45) Bekanntmachung des Hinweises auf die
Patenterteilung:
**25.01.95 Patentblatt 95/04**

(84) Benannte Vertragsstaaten:
**AT BE CH DE ES FR GB IT LI LU NL SE**

(56) Entgegenhaltungen:
**US-A- 4 645 503**

(73) Patentinhaber: **Henkel Kommanditgesellschaft auf Aktien**

**D-40191 Düsseldorf (DE)**

(72) Erfinder: **Ritter, Wolfgang, Dr.**
**Am Bandenfeld 74**
**D-5657 Haan (DE)**
Erfinder: **Pittermann, Wolfgang, Dr.**
**Schwarzbachstrasse 33**
**D-4000 Düsseldorf 12 (DE)**

(74) Vertreter: **von Kreisler, Alek, Dipl.-Chem. et al**
**Patentanwälte**
**von Kreisler-Selting-Werner**
**Postfach 10 22 41**
**D-50462 Köln (DE)**

**Beschreibung**

Die US-PS-4 645 503 beschreibt ein thermoplastisches Füllmaterial zur Implantation in lebende Knochen, das als Gemisch aus etwa 65 - 95 % eines harten feinteiligen Füllmaterials - insbesondere Hydroxylapatit - und 35 - 5 % eines bei Körpertemperatur wenigstens hinreichend festen Polymerharzes als Bindemittel ausgebildet ist. Geeignete Bindemittel sind körperresorbierbare Harze der Art Polyglycolsäure, Polymilchsäure und verwandte Verbindungen mit Molgewichten im Bereich von 400 - 5000 Dalton.

Gegenstand der offengelegten deutschen Patentanmeldung DE-A-3 229 540 sind vom lebenden Organismus resorbierbare Wachse zur mechanischen Blutstillung an körpereigenem Hartgewebe, insbesondere an Knochen, die sich dadurch auszeichnen, daß sie aus bei Körpertemperatur zähviskosen bis festen wachsartigen Polyester-Oligomeren von niederen Hydroxycarbonsäuren bestehen. Aufgrund ihrer Struktur sind diese Wachse durch körpereigene Stoffwechselmechanismen abbaubar, wobei die Geschwindigkeit des Abbaus in an sich bekannter Weise einstellbar ist. Die bevorzugten Wachse weisen mittlere Molekulargewichte im Bereich von etwa 200 bis 1.500 und insbesondere im Bereich von etwa 300 bis 1.000 auf.

Als geeignet sind insbesondere entsprechende Polyester-Oligomere aus Milchsäure und/oder Glykolsäure geschildert. Zur Regelung des mittleren Molekulargewichts dieser Polyester-Oligomeren schlägt die genannte Offenlegungsschrift vor, monofunktionelle und/oder difunktionelle Alkohole oder Carbonsäuren bzw. Carbonsäure-Anhydride und/oder primäre bzw. sekundäre Monoamine mitzuverwenden. In an sich bekannter Weise kann dann durch Wahl geeigneter Mischungsverhältnisse an Oxycarbonsäure und zusätzlicher monofunktioneller bzw. difunktioneller Komponente ein sich letztlich einstellendes mittleres Molekulargewicht vorherbestimmt werden. Die dabei anfallenden Reaktionsprodukte sind bekanntlich bezüglich ihres Oligomerisationsgrades nicht einheitlich, sie enthalten auch noch gewisse Anteile an eingesetzten Ausgangskomponenten. Im Rahmen der Optimierung resorbierbarer Wachse zur mechanischen Blutstillung an körpereigenem Hartgewebe der geschilderten Art hat sich jetzt gezeigt, daß besonders körper- und insbesondere gewebeverträgliche Wachse dann erhalten werden, wenn - unter Wahrung der allgemeinen Gesetzmäßigkeiten aus der genannten veröffentlichten deutschen Schutzrechtsanmeldung - zur Einstellung des mittleren Molekulargewichts ein ganz bestimmter dreifunktioneller Alkohol, nämlich Glycerin, eingesetzt wird. Die Kombination von Glycerin mit Oligoestern der Milchsäure und/oder der Glykolsäure führt zu abbaubaren wachsartigen Komponenten der genannten Art, die sich bei der Implantation in lebendes Körpergewebe durch eine besonders gut ausgeprägte Körperverträglichkeit auszeichnen.

Wachse dieser Art sind in der älteren - nicht vorveröffentlichten - EP-A-0 250 994 der Anmelderin als Träger bzw. Trägerbestandteil von neuen Mitteln zur Abdeckung unverletzter und/oder verletzter Bereiche menschlicher oder tierischer Haut beschrieben. Die Implantation dieser Mittel in den lebenden Körper im Sinne ihrer Verwendung als Hilfsmittel zur mechanischen Blutstillung an körpereigenem Hartgewebe ist nicht Gegenstand dieser älteren Anmeldung.

Die mit den hier betroffenen Materialien durchgeführten Untersuchungen haben weiterhin gezeigt, daß unerwünschte Gewebsschädigungen dann besonders sicher vermieden werden können, wenn durch die Herstellung und eine anschließende Reinigung des abbaubaren Wachses sichergestellt wird, daß dessen Gehalt an nicht reagierten Carboxylgruppen zumindest stark abgesenkt oder besser noch praktisch vollständig beseitigt wird. Solche freien Carboxylgruppen können auch noch dann im Reaktionsgemisch vorliegen, wenn auf die Mitverwendung von molekulargewichtsregelnden Dicarbonsäuren im Sinne des erwähnten vorveröffentlichten Vorschlages der Anmelderin verzichtet wird. Die statistische Molekulargewichts-Verteilung, die sich unmittelbar aus der Herstellung der Wachse ergibt, läßt noch immer einen gewissen Anteil an freien Carboxylgruppen, die zumindest vorwiegend als freie, monomere Hydroxycarbonsäuren vorliegen, im oligomeren Reaktionsgemisch zurück, auch dann, wenn an sich der gewünschte mittlere Zahlenwert für das Molekulargewicht eingestellt ist.

Gegenstand der vorliegenden Erfindung sind körperresorbierbare Wachse aus bei Körpertemperatur zähviskosen bis festen wachsartigen Polyester-Oligomeren der Milchsäure und/oder Glycolsäure, hergestellt unter Mitverwendung von Glycerin als polyfunktionellem Alkohol zur Regelung des mittleren Molekulargewichts der Polyester-Oligomeren auf Werte im Bereich von etwa 200 - 1500, wobei diese Wachse zusätzlich dadurch gekennzeichnet sind, daß für die Verwendung zur mechanischen Blutstillung an körpereigenem Gewebe, insbesondere Knochen, ihr Gehalt an nicht-reagierten Hydroxycarbonsäuren auf Restgehalte unter 0,5 Gew.-% abgesenkt ist.

In einer besonders bevorzugten Ausführungsform der Erfindung ist der Gehalt an nicht reagierten Hydroxycarbonsäuren auf Restgehalte unter etwa 0,2 Gewichtsprozent abgesenkt. In der Regel werden die Restgehalte an nicht reagierten Hydroxycarbonsäuren in den zum Einsatz kommenden Wachsen bei oder unterhalb von 0,1 Gewichtsprozent liegen.

Im übrigen gelten weitgehend die Angaben der genannten DE-A 3 229 540 zur Beschaffenheit und den Parametern der Wachse. So sind auch die erfindungsgemäßen Polyester-Oligomere durch ein mittleres Molekulargewicht im Bereich von etwa 200 bis 1.500 und vorzugsweise im Bereich von etwa 300 bis 1.000 gekennzeichnet. Die resorbierbaren Wachse sind bei Körpertemperatur pastös bis weich verstreichbare Materialien, die auch durch kurzzeitiges Erwärmen auf Temperaturen bis etwa 100 °C oder vorzugsweise bis etwa 60 °C in einen noch besser verstreichbaren Zustand gebracht werden können. In dieser Form eignen sie sich insbesondere zur mechanischen Blutstillung durch an sich bekannten Auftrag auf körpereigenes Gewebe, beispielsweise am verletzten oder sonstwie eröffneten Knochen.

Die Oligomer-Segmente der erfindungsgemäß beschriebenen optimierten Knochenwachse leiten sich von der Milchsäure und/oder der Glykolsäure ab. Ausgangsmaterial für die Herstellung der Oligomeren können die monomeren Hydroxycarbonsäuren der genannten Definition sein. Im allgemeinen wird man allerdings die leicht handhabbaren Dimerisationsprodukte, d. h. das Lactid und/oder das Glykolid einsetzen. Das Milchsäuredimere kann beispielsweise als L-Lactid oder auch als D, L-Lactid Verwendung finden.

Bevorzugte Wachse im Sinne der Erfindung werden erhalten, wenn man jeweils auf 1 Mol Glycerin 9 bis 10 Mol der monomeren Glykolsäure bzw. etwa 12 Mol der monomeren Milchsäure einsetzt.

Einzelheiten zur Durchführung der Oligomerisierungsreaktion finden sich in der zitierten Veröffentlichung gemäß DE-A 32 29 540.

Die erfindungsgemäß bevorzugte Reinigung des primär anfallenden Oligomeren von nicht umgesetzten Komponenten bzw. Umsetzungsprodukten eines unerwünscht niederen Mol-Gewichtes kann in einfacher Weise wie folgt bewerkstelligt werden: Man vermischt das primär angefallene Edukt mit einem wassermischbaren organischen Lösungsmittel, beispielsweise mit Aceton, Methanol, Ethanol oder dergleichen und gibt die dabei angefallene Aufschlemmung anschließend in ein Lösungsmittel, das die erwünschten Oligomerfraktionen nicht löst, jedoch ein hinreichendes Lösungsmittel für nicht umgesetzte Komponenten bzw. Komponenten niedrigen Molekulargewichts ist. Als besonders geeignetes Fällungsmedium für diese zweite Verfahrensstufe hat sich Isopropanol erwiesen. Ein zweckmäßiges Reinigungsverfahren der Erfindung sieht dementsprechend vor, primär angefallenes Oligomerisationsprodukt und Lösungsmittel von der Art des Acetons, Methanols und dergleichen etwa im Verhältnis von 1:1 zu mischen und intensiv zu digerieren. Die angefallene Feststoffsuspension wird dann in das mehrfache Volumen, beispielsweise das 7 bis 12-fache an Isopropanol eingegeben und abfiltriert. Die Flüssigphase wird abgesaugt, zweckmäßig mit Isopropanol nachgewaschen und getrocknet. Es fällt ein wachsartiges Produkt der gewünschten Qualifikation an, das praktisch frei ist von freien Carboxylgruppen.

Die hier gegebene Arbeitsanweisung zur Abtrennung unerwünschter Bestandteile der angegebenen Art aus dem primär anfallenden Oligoester ist lediglich beispielhaft zu verstehen. Sie kann im Rahmen des Fachwissens variiert werden.

**Beispiele**

Beispiele 1 bis 5

Allgemeine Vorschrift zur Herstellung der Umsetzungsprodukte von Glycolsäure mit Glycerin

In einem Dreihalskolben mit Rührer und Destillationsbrücke werden Glycolsäure und Glyzerin vorgelegt. Unter Stickstoff wird schnell auf 150 °C und dann im Verlauf von 6 Stunden von 150 auf 200 °C hochgeheizt. Dabei spaltet sich bereits der größte Anteil des Reaktionswassers, der den Umsatz der Esterkondensation anzeigt, ab. Man läßt den Ansatz auf etwa 150 °C abkühlen, evakuiert vorsichtig auf 10 Torr und vervollständigt den Umsatz bei 200 °C und 10 Torr. Nach 30 Minuten wird das Produkt bei ca. 150 °C heiß abgefüllt. Die Zusammensetzung der Ansätze und die Oligomereigenschaften sind der Tabelle 1 (Beispiel 1 bis 5) zu entnehmen.

## T a b e l l e 1

Oligohydroxycarbonsäuren aus Glycolsäure und Glycerin

| Beispiel | Edukte | | Ausbeute | Viskosität | Beschaffenheit |
|---|---|---|---|---|---|
| | Glycolsäure mol | Glycerin mol | Reaktions- wasser % | bei Meßtemperatur | |
| 1 | 8 | 1 | 100 | 2450 mPas/65-70 °C | hochviskos, leicht gelb |
| 2 | 9 | 1 | 99,1 | 3950 mPas/65-70 °C | weich, pastenartig, gelblich |
| 3 | 10 | 1 | 97 | - | hart, wachsartig, gelblich |
| 4 | 12 | 1 | 91,4 | - | hart, weiß |
| 5 | 20 | 1 | 100 | - | hart, weiß |

EP 0 290 983 B1

Beispiele 6 bis 8

Allgemeine Vorschrift zur Herstellung der Umsetzungsprodukte von Glycolid mit Glycerin

Glycolid und Glycerin wurden in einer üblichen Laborapparatur unter Stickstoff und unter Rühren innerhalb von einer Stunde auf 195 °C erwärmt. Man ließ dann 3 Stunden bei 195 °C reagieren und füllte heiß ab. Als Katalysator war eine Sn-II-Chlorid-Lösung in Ether zugesetzt (7 ml einer Lösung von 2,5 g $SnCl_2$ in 1000 ml Ether bei der Umsetzung von 3 mol Glycolid mit einem mol Glycerin).

## T a b e l l e 2

Oligohydroxycarbonsäuren aus Glycerin und Glycolid

| Beispiel | Edukte | | Beschaffenheit | Gehalt an freier Glycolsäure |
|---|---|---|---|---|
| | Glycerin mol | Glycolid mol | | Gew.-% |
| 6 | 1 | 4 | weich, wachsartig | 0,05 % |
| 7 | 1 | 4,5 | hart, kaum wachsartig | - |
| 8 | 1 | 5 | sehr hart, nicht wachsartig | - |

EP 0 290 983 B1

EP 0 290 983 B1

Beispiel 9 bis 16

Allgemeine Vorschrift zur Herstellung der Umsetzungsprodukte von Lactid mit Glycerin

Lactid (L(-)-Lactid N, B der Firma Böhringer Ingelheim) und Glycerin wurden in einer üblichen Laborapparatur unter Stickstoff und unter Rühren innerhalb von einer Stunde auf 195 °C erwärmt. Man ließ dann 3 Stunden bei 195 °C reagieren und füllte heiß ab. Als Katalysator war eine Sn-II-Chlorid-Lösung in Ether zugesetzt (7 ml einer Lösung von 2,5 g SnCl$_2$ in 1000 ml Ether bei der Umsetzung von 3 mol Lactid mit einem mol Glycerin).

Tabelle 3

Oligohydroxycarbonsäuren aus Glycerin und Lactid

| Beispiel | Edukte | | Beschaffenheit | Viskosität bei | | | Gehalt an freier Glycol- |
| | Glycerin | Lactid | | Meßtemperatur | | | säure (Gew.-%) |
| | mol | mol | | | | | |
| 9 | 1 | 2 | viskos, klar | 200 | mPas/65-70 °C | | – |
| 10 | 1 | 3 | hochviskos, klar | 850 | mPas/65-70 °C | | – |
| 11 | 1 | 4 | weich, klar | 2300 | mPas/65-70 °C | | – |
| 12 | 1 | 5 | weich, klar | 2500 | mPas/65-70 °C | | 0,125 % |
| 13 | 1 | 6 | hart, formbar, klar | 4750 | mPas/65-70 °C | | 0,075 % |
| 14 | 1 | 8 | fest, spröde, klar | 6000 | mPas/65-70 °C | | – |
| 15 | 1 | 10 | fest, spröde, klar | 14000 | mPas/65-70 °C | | – |
| 16 | 1 | 20 | fest, spröde, klar | nicht meßbar | | | – |

EP 0 290 983 B1

Beispiele 17 bis 19

Reinigung der Knochenwachse

Zur Reinigung von freier Glycolsäure und weiteren niedermolekularen Bestandteilen wurden die Knochenwachse nach Beispielen 2 bis 4 durch Umfällen nachgereinigt.

Verfahrensweise

Die Knochenwachse auf Glycolsäurebasis entsprechend der Beispiele 2 bis 4 wurden im gleichen Volumenanteil Aceton aufgeschlämmt und anschließend tropfenweise in die 10-fache Menge Isopropanol gefällt. Nach der Isolierung erfolgte eine Trocknung über 24 Stunden im Vakuumtrockenschrank bei 50 °C.

| Beispiel | Ungereinigtes Wachs nach Beispiel | Ausbeute nach Trocknung Gew.-% | Aussehen | Gehalt an freier Glycolsäure Gew.-% |
|---|---|---|---|---|
| 17 | 2 | 72,2 % | weich-pastenartig | 0,04 |
| 18 | 3 | 78 % | verformbar, wachsartig | – |
| 19 | 4 | 66 % | kaum wachs-artig, hart | – |

**Patentansprüche**

1. Körperresorbierbare Wachse aus bei Körpertemperatur zähviskosen bis festen wachsartigen Polyester-Oligomeren der Milchsäure und/oder Glycolsäure, hergestellt unter Mitverwendung von Glycerin als polyfunktionellem Alkohol zur Regelung des mittleren Molekulargewichts der Polyester-Oligomeren auf Werte im Bereich von 200 bis 1500 dadurch gekennzeichnet, daß für die Verwendung zur mechanischen Blutstillung an körpereigenem Gewebe, insbesondere Knochen, ihr Gehalt an nicht-reagierten Hydroxycarbonsäuren auf Restgehalte unter 0,5 Gewichtsprozent abgesenkt ist.

2. Resorbierbare Wachse nach Anspruch 1 dadurch gekennzeichnet, daß das wachsartige Material von seinem Gehalt an nicht umgesetzten Ausgangskomponenten wenigsten weitgehend befreit ist, wobei insbesondere der Restgehalt an Hydroxycarbonsäuren auf Werte unter 0,2 Gewichtsprozent abgesenkt ist.

3. Resorbierbare Wachse nach Anspruch 1 dadurch gekennzeichnet, daß das Polyester-Oligomere ein mittleres Molekulargewicht im Bereich von 300 bis 1000 besitzt.

4. Resorbierbare Wachse nach den Ansprüchen 1 - 3 dadurch gekennzeichnet, daß sie bei Körpertemperatur pastös bis weich verstreichbar sind oder durch kurzzeitiges Erwärmen auf Temperaturen bis 100 °C, vorzugsweise bis 60 °C in einen pastös verstreichbaren Zustand gebracht werden können.

5. Verwendung der körperresorbierbaren Wachse nach Ansprüchen 1 - 4 bei der mechanischen Blutstillung an körpereigenem Gewebe, isbesondere an Knochen.

## Claims

1. Body-resorbable waxes comprising polyester oligomers of lactic acid and/or glycolic acid, which oligomers are from viscous to solid wax-like at body temperature and have been prepared with the concomitant use of glycerol as poly-functional alcohol for regulating the average molecular weight of the polyester oligomers to values within the range of from 200 to 1,500, characterized in that, for use for the mechanical stanching of blood in endogenous body tissue, more especially bones, their un-reacted hydroxycarboxylic acid contents have been lowered to residual contents of below 0.5% by weight.

2. The resorbable waxes according to claim 1, characterized in that the wax-like material has been at least largely freed from its contents of unreacted starting components, while the residual contents of hydroxycarboxylic acids has been lowered to values of below 0.2% by weight.

3. The resorbable waxes according to claim 1, characterized in that the polyester oligomer has an average molecular weight of from 300 to 1,000.

4. The resorbable waxes according to 1 to 3, characterized in that they are from paste-like to soft-spreadable at body temperature or may be brought into a form in which they spread like a paste by brief heating to temperatures of up to 100 °C and preferably to temperatures of up to 60 °C.

5. Use of the resorbable waxes according to claims 1 to 4 for the mechanical stanching of blood in endogenous body tissue, more especially in bones.

## Revendications

1. Cires résorbables dans le corps composé d'oligomères de polyesters cireux très visqueux à solides à la température du corps obtenus à partir d'acide lactique et/ou d'acide glycolique préparées par réaction de glycérine comme alcool polyfonctionnel pour ajuster le poids moléculaire moyen des oligomères de polyester à des valeurs comprises dans l'intervalle de 200 à 1500, caractérisées en ce que pour l'utilisation en hémostase mécanique du tissu corporel notamment les os, leur teneur résiduel en acides hydrocarboxyliques qui n'ont pas réagi est abaissée en dessous de 0,5 pour-cent en poids.

2. Cires résorbables selon la revendication 1, caractérisées en ce qu'on élimine du matériau cireux, sa teneur en composants de départ qui n'ont pas réagi, au moins largement en abaissant la teneur résiduelle en acides hydroxycarboxyliques à des valeurs inférieures à 0,2 pourcent en poids.

3. Cires résorbables selon la revendication 1, caractérisées en ce que l'oligomère de polyester a un poids moléculaire moyen compris entre 300 et 1000.

4. Cires résorbables selon les revendications 1-3, caractérisées en ce qu'à la température du corps, on peut les étaler sous forme pâteuse à molle ou qu'on peut les transformer à un état pâteux pour les étaler par chauffage bref à des températures inférieures à 100°C, de préférence inférieures à 60°C.

5. Utilisation des cires résorbables dans le corps selon les revendications 1-4 pour l'hémostase mécanique des tissus corporels, notamment des os.